# EUROPÄISCHE PATENTSCHRIFT

(11) **EP 0 859 626 B1**
(45) Veröffentlichungstag und Bekanntmachung des Hinweises auf die Patenterteilung: **16.01.2002**
(21) Anmeldenummer: 96925692.4
(22) Anmeldetag: 09.07.1996
(51) Int. Cl.: A61K 38/13, A61K 9/107, A61K 9/48

(54) **CYCLOSPRORIN(E) ENTHALTENDE PHARMAZEUTISCHE ZUBEREITUNG ZUR ORALEN APPLIKATION UND VERFAHREN ZU IHRER HERSTELLUNG**
PHARMACEUTICAL PREPARATION CONTAINING CYCLOSPORIN(S) FOR ORAL ADMINISTRATION AND PROCESS FOR PRODUCING THE SAID PREPARATION
PREPARATION PHARMACEUTIQUE CONTENANT UNE CYCLOSPORINE/DES CYCLOSPORINES POUR ADMINISTRATION ORALE, ET SON PROCEDE DE PRODUCTION

(30) Priorität: 04.10.1995 DE 19537012
(43) Veröffentlichungstag der Anmeldung: 26.08.1998
(73) Patentinhaber: CicloMulsion AG, 76229 Karlsruhe (DE)
(72) Erfinder: Dietl, Hans, 83043 Bad Aibling (DE)
(74) Vertreter: Behnisch, Werner, Dr.
(86) Internationale Anmeldenummer: EP9603001
(87) Internationale Veröffentlichungsnummer: WO9712626

(56) Entgegenhaltungen:
- EP-A- 0 651 995
- DATABASE WPI Section Ch, Week 9243 Derwent Publications Ltd., London, GB; Class B05, AN 92-352740 XP002025108 & JP 04 253 907 A (GREEN CROSS CORP.) , 9.September 1992

## Beschreibung

Diese Erfindung betrifft eine neue, Cyclosporin(e) enthaltende pharmazeutische Zubereitung zur oralen Applikation, ein Herstellungsverfahren für diese pharmazeutische Zubereitung und ihre Verwendung zur oralen Applikation.

Cyclosporine sind von Wissenschaftlern der Sandoz AG, Basel entdeckte cyclische Oligopeptide aus niederen Pilzen. Insbesondere Cyclosporin A bzw. Cyclosporin G werden als Immunsupressiva, insbesondere bei Organ-Transplantationen, eingesetzt. Weiterhin bevorzugt wird das Cyclosporin-Derivat "SDZ IMM 125", ein Hydroxyethylderivat von D-Serin-8-Cyclosporin. Auch die Verwendung bei anderen Erkrankungen, z. B. Diabetes und Psoriasis sowie zahlreichen Autoimmunerkrankungen (z. B. rheumatische Arthritis, endogene Uveitis etc. ), wurde beschrieben.

Aus Pilzen wird Cyclosporin A durch Säulenchromatographie über Kieselgel als weißes amorphes Pulver gewonnen, es kristallisiert aus Aceton in weißen Nadeln mit einem Schmelzpunkt von 148 - 151°C. Neben dem Cyclosporin A sind zahlreiche andere Cyclosporine bekannt, die von Cyclosporin A bis Cyclosporin Z reichen (Römpps Chemie Lexikon, 9. Auflage, Seiten 841 - 843). Auch halbsynthetische Derivate des Cyclosporins sind bekannt und erfindungsgemäß einsetzbar. Es handelt sich dabei um chemisch einander sehr ähnliche Substanzen, die aus cyclischen Polypeptiden aus 11 zum Teil methylierten Aminsäuren bestehen. Cyclosporine sind löslich in Alkohol, Ether, Aceton und chlorierten Kohlenwasserstoffen und natürlichen Ölen (Triglyceriden von Fettsäuren).

Cyclosporin A ist zur oralen Anwendung in Kapseln zum Einnehmen sowie als Lösung zum Einnehmen im Verkehr. Dabei ist bei beiden Darreichungsformen das Cyclosporin gelöst in einer Mischung von Ethanol mit einem Pflanzenöl (Pharmakopoeia Martindale, 29th Edition, US Pharm. XXII, 619 sowie Fachinformation Sandimmun der Fa. Sandoz).

Neben Ethanol und Pflanzenöl (bevorzugt Maiskeimöl) werden zudem weitere Hilfsstoffe verwendet, um Cyclosporin A in Lösung bringen und zu halten, z. B. Poly(oxyethylen)-6-glycerol-tri(oleat, linolat). Die Verwendung dieser Hilfsstoffe zeigt die große Problematik, bei einer oralen Anwendung Cyclosporin in einer Form zu verabreichen, die eine zumindest teilweise Resorption gewährleistet.

Auch die zur Erzielung einer besseren Löslichkeit der Cyclosporine verwendeten Poly(oxyethylen)-6-glycerol-tri-oleate oder - linolate können unerwünschte Wirkungen haben, da sie nicht nur die Resorption der Cyclosporine, sondern auch die von anderen Stoffen, z. B. Fetten, Paraffinen, Vitaminen etc., beeinflussen. Zudem sollten die verwendeten Mengen an diesen Hilfsstoffen 25 mg pro kg Körpergewicht nicht überschreiten. Außerdem können diese Substanzen unerwünschte allergische Reaktionen, die bis hin zu Schockreaktionen gehen können, auslösen. Das größte Problem der oben beschriebenen Applikationsformen von Cyclosporin(en) zur oralen Anwendung ist die schlechte und zudem stark schwankende Resorption von Cyclosporin. Die Resorption ist unvollständig und variiert stark von Patient zu Patient und sogar beim gleichen Patienten von Tag zu Tag; sie beträgt im allgemeinen 20 und 50 % der verabreichten Dosis von Cyclosporin(en). Eine gleichmäßige und einheitliche Resorption ist jedoch äußerst wünschenswert. Zudem wäre es sinnvoll, Cyclosporin-Zubereitungen zur Verfügung zu haben, die insgesamt eine bessere Resorption, d. h. eine höhere Bioverfügbarkeit, gewährleisten.
Daher wurden Darreichungsformen entwickelt, bei denen Cyclosporin in einem Gemisch enthaltend Mono,- Di,- Triglyceride von Fettsäuren, Alkohol, Propylenglykol und Macrogol-Glycerol-Hydroxystearat gelöst wurde. Dies ermöglicht zwar eine bessere und gleichmäßigere Resorption, allerdings gibt es Patienten, die gegen die synthetischen Hilfsstoffe Propylenglykol und Macrogol-Glycerol-Hydroxystearat mit Überempfindlichkeitsreaktionen reagieren.
Daher wurden oral einzunehmende Cyclosporin-haltige Emulsionen entwickelt, welche nur natürliche Hilfsstoffe enthalten, nämlich neben Wasser natürliche Fette, Lecithine und Alkalisalze freier Fettsäuren (Deutsche Offenlegung P4338086.7).
Diese Emulsionen gewährleisten eine verbesserte und gleichmäßigere Resorption von Cyclosporin(en), und sie enthalten nur pharmazeutische Hilfsstoffe aus natürlichen Rohstoffen wie natürlichen Fetten, Lecithinen und Alkalisalzen von Speisefettsäuren, haben jedoch den Nachteil, daß sie im wesentlichen nur in Form einer Trink-Lösung verabreicht werden können. Ihre Verabreichung in Kapseln, insbesondere Weichgelatine-Kapseln, ist nicht oder nur unter beträchtlichem Aufwand möglich, da die Öl in Wasser Emulsion die Kapselhülle angreifen kann.

Eine Aufgabe der vorliegenden Erfindung ist es daher, eine Cyclosporin-haltige pharmazeutische Darreichungsform zur Verfügung zu stellen, die eine gute und gleichmäßige Resorption von Cyclosporin(en) ermöglicht, die keine unphysiologischen pharmazeutischen Hilfsstoffe enthält und die sowohl als Trinklösung wie auch in Kapselform verabreicht werden kann.

Die erfindungsgemäße Darreichungsform enthält:
(A) Cyclosporin(e)
(B) Monoglyceride und/oder Diglyceride und/oder Triglyceride natürlicher Fettsäuren (= Speisefettsäuren)
(C) natürliche Fettsäuren (= Speisefettsäuren) und/oder Alkalisalze natürlicher Fettsäuren
(D) 3-sn-Phosphatidylcholine und/oder Phosphatidylethanolamine

Zusätzlich kann die erfindungsgemäße Darreichungsform noch natürliche Gallensäuren und/oder deren Salze enthalten, z. B. Glykocholsäure, Tauroglykocholsäure und/oder Natriumglykolat, Tauroglykocholsäure Natriumsalz.

Um die Löslichkeit von Cyclosporin(en) in dem erfindungsgemäßen Gemisch zu erhöhen, kann dieses auch noch einwertige und/oder mehrwertige Alkohole enthalten, z. B. Ethanol und/oder Glycerin. Das erfindungsgemäße Gemisch kann als solches oder nach Verdünnung mit einer Flüssigkeit wie Wasser verabreicht werden, jedoch auch in einer Kapsel, insbesondere in Form einer Weichgelatine-Kapsel. Um einen Angriff der Magensäure zu vermeiden, kann das erfindungsgemäße Gemisch auch in Form einer magensaftresistenten, jedoch dünndarmlöslichen Kapsel, bevorzugt in einer Weichgelatine-Kapsel appliziert werden.
Als Cyclosporine können natürliche und synthetische Cyclosporine, beispielsweise die bekannten Cyclosporine A - Z, verwendet werden; bevorzugt sind Cyclosporin A und Cyclosporin G sowie das Cyclosporin-Derivat SDZ IMM 125.
Als Mono-, Di- und Triglyceride natürlicher Fettsäuren (= Speisefettsäuren) können die Glyceride natürlicher Fette eingesetzt werden, z. B. Sojaöl, Distelöl, Kokosöl (MCT-Öle), Fischöle, Maisöl, Olivenöl, Sonnenblumenöl.
Es können sowohl Mono- wie Di- wie Triglyceride in reiner Form sowie als Gemisch eingesetzt werden, z. B. eine reines Maisöl (= Triglycerid) als auch eine Gemisch, bestehend aus Mono-, Di- und Trigylceriden von Maisöl.
Bevorzugt sind Gemische, die Mono-, Di- und Triglyceride natürlicher Fettsäuren enthalten. Insbesondere bevorzugt sind Gemische natürlicher Fettsäuren (= Speise-Fettsäuren), die
20 - 60 % Triglyceride
15 - 50 % Diglyceride
und 3 - 20 % Monoglyceride enthalten.

Als freie Fettsäuren bzw. Alkalisalze von freien Fettsäuren (= Speisefettsäuren) können beispielsweise verwendet werden Myristinsäure und/oder Palmitinsäure und/oder Stearinsäure und/oder Ölsäure und/oder Linolsäure und/oder Linolensäure etc., deren Natrium- und/oder Kaliumsalze oder Gemische derselben. Die eingesetzten Mengen betragen ca. 0,2 - 25 %, bezogen auf die Gesamtmenge an Mono-, Di- und Triglyceriden, bevorzugt sind
0,5 - 10 %.
Die Alkalisalze sind gegenüber den freien Fettsäuren bevorzugt.

3-sn-Phosphatidylcholine und/oder Phosphatidylethanolamine können in reiner Form oder als Gemisch verwendet werden. Bevorzugt sind Lecithine, die sowohl Phosphatidylcholine wie auch Phosphatidylethanolamine enthalten, z. B. Lecithine aus Soja und/oder Ei.
Geeignet sind vor allem Lecithine mit einem Gehalt von mehr als 50 % 3-sn-Phosphatidylcholin und/oder teilhydriertem Phosphatidylcholin und/oder hydriertem Phosphatidylcholin.
Der Gehalt an Lecithinen beträgt üblicherweise und bevorzugt 3 - 25 Gewichts-%, bezogen auf das Gesamtgewicht des erfindungsgemäßen Gemisches.
Natürliche Gallensäuren und/oder deren Salze verbessern noch weiter die Resorption von Cyclosporin(en) aus dem erfindungsgemäßen Gemisch. Dieser Effekt ist besonders ausgeprägt, falls durch bestimmte Erkrankungen der betreffende Patient keine oder zu wenig körpereigene Gallensäuren produziert.
Um eine möglicherweise gewünschte noch höhere Löslichkeit des(r) Cyclosporins(e) zu erreichen, können zusätzlich natürliche Alkohole verwendet werden, insbesondere Ethylalkohol und/oder Glycerin in Konzentrationen bis zu ungefähr 30 Gewichts-% des Gesamtgemisches.

Wird das erfindungsgemäße Gemisch mit Wasser gemischt und leicht geschüttelt oder leicht gerührt, z. B. mit einem Löffel in einem Glas, so bildet sich sofort eine milchig aussehende Emulsion mit Tröpfchen im Durchmesser von ca. 5 µm bis ca. 100 µm. Eine vergleichbare Emulsion bildet sich, falls das Gemisch in einer Weichgelatine-Kapsel dargereicht wird und das erfindungsgemäße Gemisch im Magen-Darm-Trakt mit Wasser in Berührung kommt.
Um das saure Milieu des Magens zu vermeiden, kann es günstig sein, das erfindungsgemäße Gemisch in einer magensaft-resistenten, dünndarmlöslichen Kapsel, bevorzugt einer Weichgelatine-Kapsel, zu verabreichen. Dadurch wird das erfindungsgemäße Gemisch direkt am Ort der Resorption, nämlich dem Dünndarm, freigesetzt.
Durch die Bildung einer Mikroemulsion wie eine bessere und gleichmäßigere Resorption des Cyclosporins erreicht.
Die Resorption ist dabei nicht nur erhöht, sondern vor allem gleichmäßiger und weitgehend unabhängig von den Schwankungen, die durch die gleichzeitige Aufnahme verschiedener Lebensmittel verursacht wird. Dadurch ist sowohl eine kostengünstigere Therapie möglich sowie auch eine genauere Steuerung der Blutspiegel an Cyclosporin.
Die folgenden Ausführungsbeispiele beschreiben die erfindungsgemäße Zubereitung und das Verfahren zu seiner Herstellung. Die Erfindung ist jedoch nicht hierauf beschränkt.

### Beispiel 1

50 g Cyclosporin A werden in 810 g eines Gemisches von Mono-, Di- und Triglyceriden, gewonnen aus Maisöl, unter Rühren bei 70°C gelöst. Es werden dann 15 g Natriumoleat und 125 g Sojalecithin zugegeben und weitergerührt.
a) Die so erhaltene Zubereitung wird unter Zugabe eines Konservierungsmittels, z. B. Natriumbenzoat, in 100 ml Fläschchen abgefüllt. 1 ml davon in 1 ml Wasser gegeben und leicht gerührt, bzw. geschüttelt ergibt sich eine milchige Emulsion.
b) Die Zubereitung wird in Mengen zu je 1 g in an sich bekannter Weise in magensaftresistenten, dünndarmlöslichen Weichgelatine-Kapseln abgefüllt.

### Beispiel 2

Das Beispiel 1 wird wiederholt, wobei zur Herstellung der erfindungsgemäßen pharmazeutischen Zubereitung 50 g Cyclosporin, 750 g eines Gemisches von Mono-, Di- und Triglyceriden, gewonnen aus Maisöl, 125 g Eilecithin, 10 g Natriumoleat und 65 g des Natriumsalzes der Tauroglykocholsäure verwendet werden.

### Beispiel 3

75 g Cyclosporin A werden unter Rühren (bei ca. 60-70°C) in 120 g Ethylalkohol und 700 g Sojaöl gelöst. Es werden dann 40 g Kaliumoleat, 50 g Sojalecithin und 15 Natriumglykocholsäure zugegeben und weitergerührt. Die so erhaltene pharmazeutische Zubereitung bildet beim Mischen mit ca. 500 g Wasser eine Mikroemulsion, bei der die Tropfengröße zwischen 10 und 100 µm liegt.

## Patentansprüche

1. Cyclosporin(e) enthaltende pharmazeutische Zubereitung zur oralen Applikation,
**dadurch gekennzeichnet,**
**daß** sie aus
(A) einem oder mehreren Cyclosporin(en),
(B) Mono- und/oder Di- und/oder Trigylceriden natürlicher Fettsäuren (= Speisefettsäuren)
(C) natürlichen Fettsäuren (= Speisefettsäuren) und/oder deren Alkalisalze, und
(D) 3-sn-Phosphatidylcholin(en) und/oder Phosphatidylethanolamin(en); und wahlweise zusätzlich
(E) natürlichen Gallensäuren und/oder deren Alkalisalze, und/oder
(F) natürlichen Alkoholen besteht.

2. Cyclosporin(e) enthaltende pharmazeutische Zubereitung nach Anspruch 1,
**dadurch gekennzeichnet,**
**daß** ein Gemisch aus Mono-, Di- und Triglyceriden von natürlichen Fettsäuren verwendet wird, das
20 - 60 Gew.% Triglyceride,
15 - 50 Gew.% Diglyceride,
3 - 20 Gew.% Monoglyceride enthält.

3. Cyclosporine (e) enthaltende pharmazeutische Zubereitung nach Anspruch 1 oder 2,
**dadurch gekennzeichnet,**
**daß** als 3-sn-Phosphatidylcholin(e) und Phosphatidylethanolamin(e) enthaltende Substanzen Lecithine natürlicher Herkunft, insbesondere Soja- und/oder Eilecithine, verwendet werden.

4. Cyclosporin(e) enthaltende pharmazeutische Zubereitung nach einem oder mehreren der vorhergehenden Ansprüche,
**dadurch gekennzeichnet,**
**daß** die Zubereitung in einer magensaftresistenten, dünndarmlöslichen Kapsel, bevorzugt eine Weichgelatine-Kapsel, enthalten ist.

5. Verfahren zur Herstellung der Cyclosporine(e) enthaltenden pharmazeutischen Zubereitung zur oralen Applikation nach einem oder mehreren der vorhergehenden Ansprüche,
**dadurch gekennzeichnet,**
**daß** die Cyclosporin(e) in der pharmazeutischen Zubereitung in an sich bekannter Weise gelöst werden.

6. Verfahren zur Herstellung der Cyclosporin(e) enthaltende pharmazeutischen Zubereitung nach Anspruch 5,
**dadurch gekennzeichnet,**
**daß** die Cyclosporine zuerst in einem natürlichen Alkohol gelöst werden.

7. Verwendung einer Zubereitung nach einem der Ansprüche 1 bis 4 zur Herstellung eines Arzneimittels zur oralen Applikation.

## Claims

1. Pharmaceutical preparation containing cyclosporin(s) for oral administration,
**characterized in that,**
said preparation consists of
(A) one or more cyclosporin(s),
(B) monoglycerides and/or diglycerides and/or triglycerides of natural fatty acids (= edible fatty acids),
(C) natural fatty acids (=edible fatty acids) and/or the alkaline salts thereof, and
(D) 3-sn-phosphatidyl choline(s) and/or phosphatidyl ethanolamine(s), and optionally also
(E) natural bile acids and/or the alkaline salts thereof, and/or
(F) natural alcohols.

2. Pharmaceutical preparation containing cyclosporin(s) according to claim 1,
**characterized in that,**
a mixture of monoglycerides, diglycerides and triglycerides of natural fatty acids is used, containing
20 - 60% by wt. of triglycerides
15 - 50% by wt. of diglycerides
3 - 20% by wt. of monoglycerides.

3. Pharmaceutical preparation containing cyclosporin(s) according to claim 1 or 2,
**characterized in that**,
as substances containing 3-sn-phosphatidyl choline(s) and phosphatidyl ethanolamine(s), lecithins of natural origin, especially soybean and/or egg lecithins are used.

4. Pharmaceutical preparation containing cyclosporin(s) according to one or more of the preceding claims,
**characterized in that**,
the preparation is contained in an enteric coated capsule being soluble in the small intestine, preferably a soft gelatin capsule.

5. Process for the production of the pharmaceutical preparation containing cyclosporin(s) for oral administration according to one or more of the preceding claims,
**characterized in that**,
the dissolution of the cyclosporin(s) in the pharmaceutical preparation is performed in a manner known per se.

6. Process for the production of the pharmaceutical preparation containing cyclosporin(s) according to claim 5,
**characterized in that**,
the cyclosporins are first dissolved in a natural alcohol.

7. Use of a preparation according to any of the claims 1 to 4 for the production of a medicament for oral administration.

## Revendications

1. Préparation pharmaceutique à usage oral contenant une ou plusieurs cyclosporines, **caractérisée en ce qu'**elle se compose :
(A) d'une ou plusieurs cyclosporines,
(B) de mono- et/ou di- et/ou triglycérides d'acides gras naturels (= acides gras alimentaires),
(C) d'acides gras naturels (= acides gras alimentaires) et/ou de sels alcalins de ceux-ci, et
(D) de 3-sn-phosphatidylcholine(s) et/ou de phosphatidyléthanolamine(s) ; ainsi que, facultativement,
(E) d'acides biliaires naturels et/ou de sels alcalins de ceux-ci, et/ou
(F) d'alcools naturels.

2. Préparation pharmaceutique contenant une ou plusieurs cyclosporines selon la revendication 1, **caractérisée en ce que** l'on utilise un mélange de mono-, di- et triglycérides d'acides gras naturels contenant :
20 à 60% en poids de triglycérides,
15 à 50% en poids de diglycérides,
3 à 20% en poids de monoglycérides.

3. Préparation pharmaceutique contenant une ou plusieurs cyclosporines selon la revendication 1 ou la revendication 2, **caractérisée en ce que** l'on utilise comme substances contenant de la ou des 3-sn-phosphatidyl choline(s) et phosphatidyléthanolamine(s) des lécithines d'origine naturelle, en particulier des lécithines de soja et/ou d'oeuf.

4. Préparation pharmaceutique contenant une ou plusieurs cyclosporines selon l'une ou plusieurs des revendications précédentes, **caractérisée en ce que** la préparation est contenue dans une gélule résistante aux sucs gastriques et soluble dans l'intestin grêle, de préférence une gélule en gélatine molle.

5. Procédé de fabrication de la préparation pharmaceutique à usage oral contenant une ou plusieurs cyclosporines selon l'une ou plusieurs des revendications précédentes, **caractérisé en ce que** la ou les cyclosporines sont dissoutes d'une manière en soi connue dans la préparation pharmaceutique.

6. Procédé de fabrication de la préparation pharmaceutique contenant une ou plusieurs cyclosporines selon la revendication 5, **caractérisé en ce que** les cyclosporines sont d'abord dissoutes dans un alcool naturel.

7. Utilisation d'une préparation selon l'une des revendications 1 à 4 pour fabriquer un médicament à usage oral.
